# EUROPEAN PATENT APPLICATION

(11) **EP 1 932 516 A1**
(43) Date of publication of application: **18.06.2008**
(21) Application number: 06077203.5
(22) Date of filing: 11.12.2006
(51) Int. Cl.: A61K 9/00, A61K 9/127, A61K 9/51, A61K 31/19, A61K 31/196, A61K 31/202, A61K 31/505, A61K 31/445, A61K 31/573, A61K 31/56, A61P 35/00, A61P 35/02

(54) **Anti-inflammatory compounds containing compositions for treatment of cancer**

(71) Applicant: Universiteit Utrecht Holding B.V., 3584 CM Utrecht (NL)
(72) Inventor: The designation of the inventor has not yet been filed
(74) Representative: van Loon, C.J.J.

(57) **Abstract**

The present invention relates to compositions comprising anti-inflammatory compounds, and especially to the use of such compositions in the treatment of cancer or in the inhibition of cancer growth. More specifically, the invention relates to a method for targeting an anti-inflammatory compound to tumor tissue.

## Description

The present invention relates to compositions comprising anti-inflammatory compounds, and especially to the use of such compositions in the treatment of cancer or in the inhibition of cancer growth. More specifically, the invention relates to a method for targeting anti-inflammatory compounds to tumor tissue. Even more specifically, the present invention aims to provide the use of such compositions in the treatment of non-lymphatic cancers, and preferably solid (non-hematological) malignancies and metastases (solid primary and secondary tumors), and is hence in the field of oncology. In the treatment of non-solid tumor or lymphatic cancer types, e.g. chronic and acute lymphatic leukaemia, Hodgkin and non-Hodgkin lymphomas, anti-inflammatory compounds can however also be used

There is a huge group of compounds having an anti-inflammatory effect. Traditionally, these compounds were divided in anti-inflammatory agents with a steroid skeleton and in non-steroid anti-inflammatory drugs (NSAID's).

Anti-inflammatory agents with a steroid skeleton act through cytosolic glucocorticoid receptors that affect genomic processes. At higher concentrations the agents can also act through non-genomic mechanisms of action.

Well-known examples of this category are the corticosteroids, such as glucocorticoids. Corticosteroids have been proposed as active ingredients in the treatment of cancer. For example, Coleman has described in Biotherapy 4(1) (1992), 37-44 that in tumor therapy, glucocorticoids are often used for their anti-inflammatory and anti-emetic potential and for the treatment of haematological malignancies due to their efficient cytolytic activity on cells of lymphoid origin.

In the treatment of lymphatic cancer types, *e.g.* chronic and acute lymphatic leukaemia, Hodgkin and non-Hodgkin lymphomas, steroids have shown antitumor activity. In view of the fact that cells of lymphatic origin can be suppressed by corticosteroids (immuno suppressive activity) this is not surprising.

An example of such a treatment is disclosed in US-A-6,090,800 wherein vesicles, liposomes and micelles are described that contain lipid soluble steroid prodrugs. It is noted in this document that "steroids such as cortisone and dexamethasone are potent immune suppressants and are used to treat conditions such as auto-immune diseases, organ transplant rejection, arthritis, skin, mucosal membrane and ophthalmic inflammation, as well as neoplastic conditions such as lymphoma". In addition, this US patent teaches targeting to IL-2 receptors on T-cells.

Further reports in the 1980's and 1990's demonstrated that glucocorticoids could also decelerate solid tumor growth. Several studies showed that this effect was not directly aimed at the tumor cells, but rather mediated by interference with the tumor vascularization (see in this light, e.g., Folkman et al. in Science 221 (4612) (1983), 719-723; Lee et al. in Cancer Res. 47(19) (1987), 5021-5024; McNatt et al. in J. Ocul. Pharmacol. Ther. 15(5) (1999) 413-423; and Crowley et al. in Oncology 45(4) (1988), 331-335).

The exact mechanism of interference is, however, unclear.

It has been suggested that it is mediated by inhibition of endothelial cell proliferation and migration (Cariou et al. in Cell. Biol. Int. Rep. 12(12) (1988), 1037-1047), basement membrane turnover (Folkman et *al. (ibid);* Ingber et al. in Endocrinology 119(4) (1986), 1768-1775), and/or inhibition of pro-angiogenic factors (like plaminogen activator and vascular endothelial growth factor) (Blei et al. in J. Cell Physiol. 155(3) (1993), 568-578; Nauck et al. in Eur. J. Pharmacol. 341(2-3) (1998), 309-315).

These anti-inflammatory compounds based on steroid structures do not form part of the present invention.

The non-steroidal anti-inflammatory agents, which do not have a steroid skeleton can inhibit the inflammatory cascade at several points:
- inhibition of the activities of the different cyclo-oxygenase (COX) enzymes (1, 2 and 3), which in turn inhibit the formation of prostaglandins (PGs);
- specific inhibition of COX-2 or COX-3 (and not COX-1) which, more specifically, inhibits pathological PG formation;
- lipoxygenase inhibition, inhibiting formation of leukotrienes;
- inhibition of leukocyte function-inhibition of endothelial cell-leukocyte interactions-inhibition/neutralization of pro-inflammatory cytokines and signalling molecules-inhibiting or stimulating the activity of regulatory cells/cytokines or signalling molecules; as well as
- at present unknown mechanisms.

The non-steroidal anti-inflammatory agents have diverse chemical structures and can be classified (non-limitative) as:
(a) salicylates (like aspirin, methyl salicylate, diflunisal, benorylate, faislamine, amoxiprin);
(b) arylalkanoic acids (like diclofenac, indometacin, sulindac, 2arylpropionic acids);
(c) profens (like carprofen, fenoprofen, flurbiprofen, ibuprofen, ketoprofen, ketorolac, loxoprofen, naproxen, tiaprofenic acid);
(d) N-arylanthranilic acids (like fenamic acids, mefenamic acid, meclofenamic acid);
(e) pyrazolidine derivatives (like phenylbutazone, oxyphenylbutazone);
(f) oxicams (like piroxicam, meloxicam);
(g) coxibs (like celecoxib, rofecoxib, valdecoxib, parecoxib, etoricoxib); sulphonanilides (like nimesulide);
(h) lipoxygenase inhibitors (like baicalein, caffeic acid, esculetin, gossypol, nordihydroguaiaretic acid, flurbiprofen, nordihydroguaiaretic acid, eicosatriynoic acid, 5-hydroxyeicosatetraenoic (HETE) lactone, 5(*S*)-HETE, eicosatetraynoic acid);
(i) macrolide derivatives (like 9-(S)-dihydroerythromycin derivatives);
(j) natural compounds with anti-inflammatory properties like genistein, kaempferol, quercetin, resorcinol, resveratrol, docosahexaenoic acid, daidzein, epigallocatechin gallate (EGCG), alpha-tocopherol, ellagic acid, chlorogenic acid, p-coumaric acid, phytic acid, ferulic acid, vanillin, cinnamic acid, hydroxycinnamic acid, anthocyanins, catechins, isoflavones, hesperetin, naringin, rutin, silymarin, tangeretin, tannins, lycopene, lutein, saponins, terpeneol, terpene limonoids, lignans, or in general compounds belonging to the classes of terpenoids (isoprenoids), polyphenolics, glucosinolates, thiosulfonates, phytosterols, anthraquinones, capsaicins, piperines, chlorophylls, betaines, pectines, or oxalic acids;
(k) anti-inflammatory peptides (antiflamins) (like peptides derived from seminal vesicle proteins, selectin-binding peptides, cationic peptides based on Bactericidal permeability increasing protein, IL-2 derived peptides);
(l) anti-inflammatory cytokines (like IL-1 receptor antagonist, IL-4, IL-6, IL-10, IL-11, and IL-13);
(m) pro-inflammatory cytokine inhibitors (like tumor necrosis factor-alpha, IL-18);
(n) galectins (like Galectin-1);
(o) antibodies neutralizing pro-inflammatory signaling molecules/cytokines, like antibodies against TNF-alpha, IL-1 *etc.;* and
(p) statins.

As to the latter class of compounds, it is noted that statins are well-known lipid lowering agents. Particularly, drugs of the statin-class are structurally similar to hydroxymethylglutaryl-coenzyme A (HMG-CoA,) a precursor of cholesterol, and, as such, these drugs are competitive inhibitors of HMG-CoA reductase. This latter enzyme regulates the rate-limiting step in the synthesis of cholesterol. The inhibition of this enzyme results in a decrease in intracellular cholesterol level, which leads to an upregulation of LDL-receptor activity and a reduction of the entry of LDL into the circulation.

Recently, Rutishauser in Swiss Med Wkly, (2006) 136 (3-4): 41-49 supplemented the effects of statin by evidence showing that statins may affect the function of immune and inflammatory cells.

Also for the non-steroid anti-inflammatory compounds the epidemiological/clinical trial data show varying results. Some studies show a reduced cancer incidence in the population and therapeutic effects in cancer patients while others report increased cancer incidence and failure of therapy. See, in this light, *e.g*. Bongartz et al. JAMA (2006) 295(19): 2275-2285; Biancone et al. Gut (2006) 55(2): 228-233; Mrozek et al. J Clin Endocrinol Metab. (2006) 91(6): 2201-2204.

However, the data on these effects in respect of cancer are conflicting or inconsistent and/or the mechanisms by which this occurs are unknown.

These conflicting studies indicate that the effects of anti-inflammatory compounds are not clear-cut and that additional measures are needed to predictably exploit the full potential of anti-inflammatory compounds as anticancer therapeutics.

In addition, the amounts of anti-inflammatory compound required to obtain any effect, if at all, are high. That is, if one were to use anti-inflammatory compounds in cancer- or tumor-therapy, one would need high dosing (typically exceeding the normal dose for such compounds by one or more orders of magnitude, that is with *e.g.* a factor 10-100 higher than the amount required for the anti inflammatory effect, prolonged periods of time to obtain significant tumor growth inhibition. This would intensify all kinds of side-effects and will most likely cause toxicities to arise. More specifically, such high doses may lead to considerable side-effects and may eliminate the important functions of the inflammatory process in response to inquires and infections elsewhere in the body.

The present inventors have found that it is the anti-inflammatory effect of the non-steroid anti-inflammatory compounds that lead to the anti-cancer activity. To make use of this finding, the non-steroid anti-inflammatory compounds need however be selectively delivered to tumor tissue to increase local drug concentrations and direct the pharmacological effect towards the cell types involved in progression of cancer.

It has now been found that a long-circulating colloidal carrier composition comprising a non-steroidal anti-inflammatory agent encapsulated in particular types of carriers, can be used to manufacture a medicament useful in the treatment of cancer.

Accordingly, the present invention relates to the use of a long-circulating colloidal carrier composition, such as liposomes, nanocapsules and polymeric micelles, comprising a natural or synthetic non-steroidal anti-inflammatory compound, for the manufacture of a medicament useful in the treatment of cancer. Compositions in accordance with the present invention were found to inhibit tumor growth, especially solid tumor growth, and can hence be used for this effect in the treatment of cancer. More specifically, the invention relates to a method for targeting active ingredients, *viz.* natural or synthetic non-steroidal anti-inflammatory agents, to tumor tissue. Even more specifically, the present invention provides the use of such compositions in the treatment of cancers, and preferably solid (non-hematological) malignancies and metastases (solid primary and secondary tumors).

The long-circulating carriers have very favorable pharmacokinetics, a favorable tissue distribution behavior and an efficient half-life. Additionally, a stable association between the natural or synthetic non-steroidal anti-inflammatory agent and the carrier system, is proposed, while the loading with the drug is efficient. Further, a good biological availability at the site where activity is required is observed. Without wishing to be bound by any theory, it is hypothesized that the carriers have an interaction with macrophages, tumor cells and/or endothelial cells in the tumor. This would mean that said cell type could liberate the encapsulated drug molecules to allow interaction with itself or other cell types in and around the tumor. As the colloidal drug carriers are to a significant extent processed by cells and/or intracellular and extracellular enzymes to liberate the encapsulated compounds, the preferred natural or synthetic non-steroidal anti-inflammatory agents should be able to resist these processing steps in order to remain therapeutically active and reach their site of activity.

For this reason, in a preferred embodiment, the non-steroid anti-inflammatory compounds useful in the present invention are compounds of the categories (a)-(j) and (p), and more preferred of the categories (a)-(i) and (p), described herein-above. In the most preferred embodiment, the non-steroid anti-inflammatory compound is selected from the group consisting of the poly(phenols) and the statins.

In a preferred embodiment of the present invention, the anti-inflammatory compound is selected from the group consisting of atorvastatin, ceruvastatin, fluvastatin, lovastatin, pravastatin, simvastatin, rosuvastatin and derivatives thereof, such as salts, solvates, esters, etc.

In the working examples, caffeic acid and pravastatin are representative, but non limiting, for the non-steroid anti-inflammatory compounds. Preferably, the long-circulating carriers used in the compositions of the invention are long-circulating liposomes. With such types of long-circulating carriers, it has been found (*vide infra*) that tumor growth can be reduced with more than 60% at a dose of only 10 mg pravastatin/kg body weight or 100 mg caffeic acid/kg body weight.

The use of long-circulating colloidal carrier compositions comprising a natural or synthetic non-steroidal anti-inflammatory agent and a carrier-forming substance, and optionally a further useful active ingredient, for the manufacture of a medicament useful in the treatment of cancer and especially of solid (non-haematological) malignancies and metastases (solid primary and secondary tumors), makes the effects of the natural or synthetic non-steroidal anti-inflammatory agents predictable, reduces the overall dose, increases tumor specificity and, hence, decreases the likelihood of side effects. This is important as the doses needed to observe the anti-cancer effects for the free drugs are comparatively high, which would intensify side-effects and could cause new toxicities to arise.

The present invention focuses on the aspect of providing measures to predictably exploit the full potential of anti-inflammatory compounds. It is, hence, an objective of the present invention to find a method to target anti-inflammatory compounds to tumor tissue for or in the treatment, retardation or inhibition of cancer.

In accordance with the present invention, it has been found that by selectively delivering non-steroid anti-inflammatory compounds to tumor tissue so that local drug concentrations are increased, a pharmacological effect towards cell types involved in the progression of cancer is achieved.

It has been found that long-circulating carriers, and especially long-circulating liposomes, nanocapsules and polymeric micelles, are capable of efficiently delivering statin/natural or synthetic non-steroidal anti-inflammatory drugs to a specific site, i.e. to a tumor. In particular, the present invention provides a medicament for or in the treatment of cancer, suitable to administer natural or synthetic non-steroidal anti-inflammatory agents, in relatively low dosages.

In order to be effective, the anti-inflammatory compounds are encapsulated in colloidal carriers. Such carriers should have a selected mean particle diameter in the size range between about 10-400 nm (preferably between 40 and 200 nm in diameter), giving an increased localization and improved retention of the encapsulated compound at the tumor site. The carrier system should provide stable encapsulation and can provide protection from degradation. Examples of such colloidal carrier system are long-circulating liposomes, lipoproteins, lipid micelles, polymeric micelles, nanoparticles and nanocapsules, which carriers should be long-circulating. Long-circulating carriers preferably have a circulation half life of at least 3 hours, and especially at least 6 hours. The circulation half life is, as the person skilled in the art appreciates, defined as the time at which the second linear phase of the logarithmic carrier, for instance liposomal, clearance profile reaches 50% of its initial concentration, which is the extrapolated plasma concentration at t=0.

Suitable long-circulating colloidal carriers have very favorable pharmacokinetics, a favorable tissue distribution behavior and an efficient half life. Additionally, a stable association between anti-inflammatory compound and the carrier system is observed, while the loading with anti-inflammatory compound is efficient. Further, a good biological availability at the site where activity is required is observed.

Without wishing to be bound by any theory, it is believed that carriers used in the present invention accumulate at sites of malignant tissues such as tumors as a result of the enhanced permeability of tumor vasculature as compared to healthy endothelium, allowing an improved localization and improved retention of the statin or natural or synthetic non-steroidal anti-inflammatory agent at these sites. It is hypothesized that the colloidal carriers have an interaction with macrophages in the tumor. This would mean that said cell type could be down regulated in tumor therapy, or that it may liberate the encapsulated anti-inflammatory compounds to allow interaction with other cell types in and around the tumor.

In a preferred embodiment the long-circulating carrier is a liposome, a nanocapsule or a polymeric micelle. Other suitable long-circulating carriers can be based on lipoproteins, and especially high density lipoproteins and low density lipoproteins, and on lipoprotein mimetics or neo-lipoproteins. In a more preferred embodiment the long-circulating colloidal carrier is a liposome, a nanocapsule or a polymeric micelle,that has a neutral or negative charge at physiological conditions.

Other suitable long-circulating carriers can be based on lipoproteins, and especially high density lipoproteins and low density lipoproteins, and on lipoprotein mimetics or neo-lipoproteins. It has been found that long-circulating microvesicles, and especially long-circulating liposomes, nanocapsules and polymeric micelles, are capable of efficiently delivering anti-inflammatory compound drugs to a specific site, *i.e.* to a tumor.

The long-circulating colloidal carriers used in accordance with the present invention typically have a mean particle diameter of less than 500, preferably less than 450, and more preferably less than 300 nm as determined by Dynamic light scattering using a Malvern 4700^{™} system equipped with a He/Ne laser, and preferably of about 40 - 200 nm. Moreover, as can be seen in the working examples (*vide infra*), the carriers of the invention have a rather small polydispersity which means that the particle size distribution is narrow. Preferably, the polydispersity, which is calculated by the software belonging to the dynamic light scattering equipment, is less than 0.25, and more preferably less than 0.2.

In the most preferred embodiment, the colloidal carrier is formed by long-circulating liposomes comprising a non-charged vesicle-forming lipid, 0-20 mole percent of an amphipathic vesicle-forming lipid derivatised with polyethyleneglycol, 0-50 mole percent of a sterol, and 0-10 mol % of a negatively charged vesicle-forming lipid, which liposomes have a selected mean particle diameter in the size range between about 40 - 200 nm.

Such long-circulating liposomes are already known in the art, but not in combination with natural or synthetic non-steroidal anti-inflammatory agents for the treatment of cancer. More particularly, these known liposome systems are described to be useful in site-specific treatment of inflammatory disorders in WO-A-02/45688. For the preparation of suitable compositions to be used in the present invention, the preparation methods described in said WO-A-02/45688 are incorporated herein by reference. In this document WO-A-02/45688, the liposome systems described in EP-A-0 662 820 are adapted to become "long-circulating".

EP-A-1 044 679 relates to liposomes having a drug included therein, which are said to have an ensured stability in blood. In addition these liposomes have an active targeting property to proteoglycan-rich areas. These areas are created because with some diseases, an over-production of proteoglycans occurs; said proteoglycans keeping cell surfaces anionic. To target liposomes to these anionic surfaces, the liposomes need to be cationic in nature. Thereto, the said liposomes require the presence of a basic compound taking positive charge within a physiological pH range.

The liposomes of the present invention do not require the active targeting property described in EP-A-1 044 679. That is, no specific homing groups are required to selectively bring the colloidal carriers containing anti-inflammatory compounds to the tumor sites. However, to increase the selectivity to an even higher extent, it is possible to attach or incorporate tumor specific antibodies or receptor ligands or food compounds at the outside surface of the carrier molecules so as to increase the interaction possibilities with the tumor cells or the cells in or around the tumor. The "targeting" of the neutral or optionally negatively charged liposomes of the present invention is ruled by the above-identified increased permeability in the tumor vasculature. That is, the present invention is for a major part based on passive accumulation, rather than active targeting.

The colloidal carriers, such as the liposomes useful in the present invention should not have a positive charge and should hence not comprise components that give the carriers a positive charge at physiological pH; that is at physiological pH, being a pH of between 6 and 8, the overall charge of the carrier to be used in the present invention should be neutral or negatively charged. Preferred liposomes are based on non-charged vesicle-forming lipids. Neutral or non-charged vesicle-forming lipids lead to a suitable long circulation time.

Typically, 5-10 mole% of negatively charged lipids may be present. Preferred lipids to be used to prepare the microvesicles used in the invention comprise saturated phospholipids and sphingolipids in combination with sterols, such as cholesterol and/or ergosterol and derivatives thereof. Substitution (complete or partial) of these basic components by e.g. sphingomyelines appeared to be possible.

To secure a suitable stability in the blood circulation system 10-50 mole% sterols should be present in the microvesicle material. Suitable liposome constituents are described in the above-identified WO-A-02/45688 and EP-A-0 662 820. More preferably, the liposomes contain at least one type of polymer lipid conjugates, such as lipids derivatised with polyalkylene glycol, preferably with polyethylene glycol (PEG). Suitable polymer-lipid-conjugates have a molecular weight of between 200 and 30,000 Dalton.

Other suitable candidates to be used in these polymer-lipid-conjugates or water-soluble polymers such as: poly ((derivatized) carbohydrate)s, water-soluble vinylpolymers (*e.g.* poly(vinylpyrrolidone), polyacrylamide and poly(acryloylmorpholine) and poly(methyl/ethyl oxazone). These polymers are coupled to the lipid through conventional anchoring molecules. Suitably, the concentration of polymer lipid conjugates is 0-20 mole%, and preferably 1-10 mole%, based upon the total molar ratio of the vesicle forming lipids. The presence of these polymer-lipid-conjugates has a favorable effect on the circulation time. However, by carefully selecting specific lipid compositions an physical specifications suitable long circulation times can be obtained without using a polymer-lipid-conjugate; for example, 50-100 nm liposomes of distearylphopshatidylcholine and cholesterol and/or sphingolipids like sphingomyelin. The liposomes may additionally contain one or more types of charged vesicle-forming lipids, e.g. phosphatidylglycerol, phosphatidylethanolamine, (di)stearylamine, phosphatidylserine, dioleoyl trimethylammonium propane, phosphatidic acids and cholesterol hemisuccinate. Typically, the concentration of charged vesicle-forming lipids is 0-15 mole%, preferably 0-10 mole% based upon the molar ratio of the vesicle forming lipids.

Polymeric micelles to be used in the present invention can be made in accordance with the method described in EP-A-1 072 617 adapted in accordance with the above-described method for the preparation of liposomes.

Passive loading of the active ingredients into the liposomes by dissolving the anti-inflammatory compounds in the aqueous phase is sufficient in order to reach an encapsulation as high as possible, but other methods can also be used.

Where in this description reference is made to charged/uncharged/amphiphatic, and so on, this reference relates to physiological conditions.

As said, the present invention also relates to pharmaceutical compositions comprising anti-inflammatory compounds, encapsulated in colloidal carriers, as defined herein-above. That is, the present invention provides a medicament for or in the treatment of cancer, suitable to administer anti-inflammatory compounds in relatively low dosages. Suitably, the medicament is a medicament for parental or local application. Application through the oral or pulmonal route are however also possible.

Water soluble statins or natural or synthetic non-steroidal anti-inflammatory agents which can be advantageously used in accordance with the present invention are alkali metal and salts prepared from statins or natural or synthetic non-steroidal anti-inflammatory agents and compounds which possess these characteristics intrinsically. If more than one group in the statin or natural or synthetic non-steroidal anti-inflammatory agent molecule is available for salt formation, mono- as well as di-salts may be useful. As alkaline metal salts the potassium, sodium, calcium, iron, zinc and ammonium salts are preferred. Also other, positively or negatively charged, derivatives of statins or natural or synthetic non-steroidal anti-inflammatory agents can be used.

Also lipophilic derivatives prepared from statins or natural or synthetic non-steroidal anti-inflammatory agents having one or more free hydroxyl groups and lipophilic aliphatic or aromatic carbon acids can be advantageously used. These compounds, when esterified with one or two alkyl carbon acids, whereby the alkyl moieties contain more than 10 C-atoms, such as palmityl and stearyl acid, are preferred.

The lipid-polymer-conjugates and compositions thereof in accordance with the present invention may be prepared by methods as known in the prior art. Passive loading of the active ingredients into the liposomes by dissolving the natural or synthetic non-steroidal anti-inflammatory agents in the aqueous phase is sufficient in order to reach an encapsulation as high as possible, but other methods can also be used. The lipid components used in forming the liposomes may be selected from a variety of vesicle-forming lipids, such as phospholipids, sphingolipids and sterols. Substitution (complete or partial) of these basic components by *e.g.* sphingomyelins and ergosterol appeared to be possible. For effective encapsulation of the, preferably water-soluble, natural or synthetic non-steroidal anti-inflammatory agents in the carriers, thereby avoiding leakage of the drug from the carriers, especially phospholipid components having saturated, rigidifying acyl chains have appeared to be useful. The beneficial effects observed after one single injection of the water soluble statin containing long-circulating liposomes according to the invention are very favorable.

In accordance with the invention, effective inhibitions in tumor growth have been observed in particular embodiments with relatively low dosages which are over an order of magnitude lower than usually reported to achieve anti-tumor effects. Generally, effects are achieved for amounts of for example 0.5-45 mg/kg body weight per week.

A composition used in accordance with the present invention may comprise one or more additional components:

Compositions to be used in accordance with the present invention may suitably contain or comprise at least one compound selected from the group consisting of cytostatic agents and cytotoxic agents, preferably at least one compound selected from the group consisting of doxorubicin and taxol.

Preferred components are cancer-therapeutics that have different mechanisms of action like glucocorticoids and corticosteroids.

In yet another aspect, the present invention is directed to pharmaceutical compositions comprising a long-circulating carrier, a statin or natural or synthetic non-steroidal anti-inflammatory agent contained therein and at least one cytostatic and/or cytotoxic agent preferably selected from the group consisting of anthracyclins (derivatives), topoisomerase I inhibitors and vinca-alkaloids.

Moreover, suitable use can be made of compositions comprising at least one component selected from the group consisting of immunomodulators and immunosuppressants. Examples of such components are methotrexate, cyclophosphamide, cyclosporin, muramyl peptides, cytokines and penicillamine.

The present invention will now be described in more detail, while referring to the following examples. In the examples, reference will be made to figures, wherein:

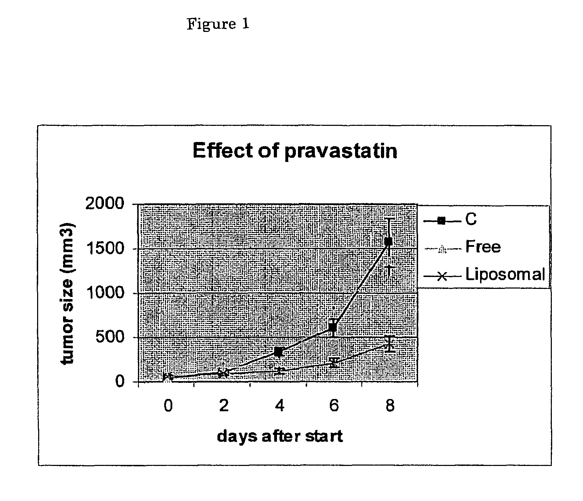

Figure 1 shows the effect of free or liposomal pravastatin on tumor growth in B16 melanoma bearing mice; mice received a single injection with the indicated dose and formulation of pravastatin on the day tumors became palpable; tumor volume 1 week later is reported; Mean ± S.D. N=5 mice/experimental group; and

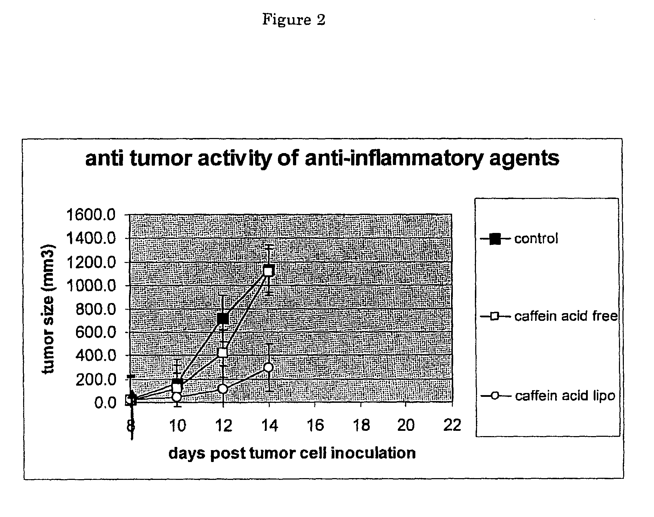

Figure 2 shows the effect of free or liposomal caffeic acid on tumor growth in B16 melanoma bearing mice; mice received a single injection with the indicated dose and formulation of caffeic acid on the day tumors became palpable; tumor volume 1 week later is reported; Mean ± S.D. N=5 mice/experimental group.

Any percentages mentioned are percentages by weight drawn to the final composition, unless otherwise indicated. In the statistical analysis of the figures, data were analysed by one-way ANOVA with Dunnett's post test using GraphPadInStat version 3.05 for Windows, GraphPad Software (San Diego, USA). Data were logarithmically transformed to correct for significant differences between SD of groups, when appropriate according to Bartlett's test.

### Example 1 - Liposome preparation

33.8 mg of egg phosphatidylcholine (EPC) (Lipoid Ludwigshafen), 9.67 mg of cholesterol (Sigma Aldrich) and 30.0 mg of poly-[N-(2-hydroxyethyl)-L-glutamine]-stearylamine (PHEG-stearylamine) (synthesised) were weighed and transferred in a 50 ml round-bottom flask. 500 kBq of tritium-labeled cholesteryloleylether was added as a lipid marker. The lipids and the label were dissolved in about 10 ml of ethanol. Thereafter, the mixture was evaporated to dryness in a Rotavapor during 1 hour under vacuum at40°C, followed by flushing with nitrogen gas during 1 hour. Phosphated buffered saline (PBS) was added to the dry lipid film and the mixture was shaken for one hour in the presence of glass beads in order to enable complete hydration of the lipid film. The liposomal suspension was transferred to an extruder (Avestin, maximum volume 15 ml) and extruded under pressure, using nitrogen gas, 6 times through 2 polycarbonate filters, one being placed on top of the other, said two filters having a pore size of 200 and 100 nm respectively, and 18 times through filters having a pore size of 100 nm and 50 nm respectively. Subsequently, the liposomal suspension was dialysed in a dialysing compartment (Slide-A-Lyzer; 10,000 MWCO) 2 times during 24 hours against 1 liter of sterilised PBS.

The mean particle size of the liposomes was determined by means of light scattering (Malvern Zeta-sizer) and was found to be 93.6 ± 0.9 nm, the polydispersity index being 0.099 ±0.02. The lipid loss during preparation of the liposomes was 25 %, determined by comparing the final radioactivity of the preparation with the activity before the extrusion procedure. The suspension of liposomes was stored in a nitrogen atmosphere at 4°C.

### Example 2

Long-circulating liposomes were prepared by dissolving dipalmitoylphosphatidylcholine (DPPC) (Lipoid GmbH, Ludwigshafen, Germany), cholesterol (Chol) (Sigma, St. Louis, USA), and poly(ethylene) glycol 2000-distearoylphosphatidylethanolamine (PEG-DSPE) (Lipoid GmbH) in a molar ratio of 1.85:1.0:0.15, respectively, in chloroform:methanol (2:1 vol:vol) in a round-bottom flask. Typically batch sizes of 1000-2000 µmol total lipid were used. Caffeic acid was dissolved together with the lipids. A lipid film was made under reduced pressure on a rotary evaporator and dried under a stream of nitrogen.

Similarly, liposomes were formed by addition of an aqueous solution of 10 mg/ml pravastatin (Sigma-Aldrich). The water-soluble statin was used to ensure stable encapsulation in the liposomes. In case of labeling of the liposomes with ¹¹¹In-oxine (Mallinckrodt Medical, Petten, The Netherlands), liposomes were formed by addition of 5 mM DTPA/10 mM Hepes/135 mM NaCl-buffer pH 7 to the lipid film, according to a procedure described by Boerman et al. in J. Nucl. Med. 36(9) (1995), 1639-1644. Liposome size was reduced by multiple extrusion steps through polycarbonate membranes (Nuclepore, Pleasanton, USA) with a final pore size of 50 nm. The pore was 400 mm in the preparation of the short- circulating liposomes. Unencapsulated material was removed by dialysis with repeated change of buffer against 10 mM Hepes/135 mM NaCl-buffer pH 7 at 4°C.

The mean particle size of the long-circulating liposomes was determined by dynamic light scattering to be 0.1 µm with a polydispersity value of approximately 0.1. The polydispersity value varies between 0 and 1. A value of 1 indicates large variations in particle size, whereas a value of 0 indicates a complete monodisperse system. Thus, the present preparations showed limited variation in particle size. The amount of lipid in the liposome dispersion was determined by colorimetric phosphate determination according to Rouser (Lipids 5 (1970), 494-496).

The concentration of pravastatin in the liposomes was determined by spectrophotometric absorbance at 238 nm; samples were measured on a Perkin Elmer UV-VIS spectrophotometer in ethanol using dissolved lipids in ethanol as controls.

### Example 3 - Tissue distribution of liposomes

### B16 murine melanoma or C26 colon carcinoma cells were cultured at

37°C in a 5% CO₂-containing humidified atmosphere in DMEM medium (Gibco, Breda, The Netherlands) containing 10% (v/v) fetal calf serum supplemented with 2mM L-glutamine, 100 IU/ml penicillin, 100zg/ml streptomycin and 0.25g/ml amphotericin B (Gibco).

Male C57B1/6 mice or male Balb/c mice (6-8 weeks of age) were obtained from Charles River, kept in standard housing with standard rodent chow and water available *ad libitum,* and a 12 h light/dark cycle. Experiments were performed according to national regulations and approved by the local animal experiments ethical committee. For tumor induction, 1 x 10⁶ B16 melanoma cells were inoculated subcutaneously in the flank of syngeneic C57BI/6 mice, whereas 1 x 10exp6 C26 tumor cells were inoculated in Balb/c mice.

The tissue distribution of ¹¹¹In-labeled PEG-liposomes according to Example 1 in tumor bearing mice was determined as follows. At a tumor volume of approximately 1 cm³, mice were injected *i.v.* with 25 µmol lipid/kg (corresponding to 30 x 10⁶ cpm/mouse) of ¹¹¹In-labeled liposomes. At 6 h and 24 h after injection animals were sacrificed, a blood sample was taken and tumor, lung, liver, spleen and kidneys were dissected, weighed and radioactivity was counted. The results are shown in Table 1.

**Table 1. Tissue distribution of long-circulating liposomes at 6 h and 24 h after intravenous administration in s. c. C26 colon carcinoma or s. c. B16F10 melanoma bearing Balb/c and C57Bl/6 mice, respectively. Tumors weighed approximately 1 g. Values represent % of injected dose/ g tissue.**

| **Organ** | **C26 colon carcinoma** | | | **B16F10 melanoma** | |
|---|---|---|---|---|---|
| | **6h** | **24h** | | **6h** | **24h** |
| **blood** | 21.9±4.5 | 7.1±2.7 | | 24.2±2.3 | 6.3±0.7 |
| **tumor** | 5.1±1.9 | 7.4±2.2 | | 5.3±2.9 | 5.8±1.5 |
| **lung** | 9.5±2.7 | 3.6±0.6 | | 12.1±0.7 | 3.2±1.3 |
| **spleen** | 23.8±11.3 | 25.9±10.1 | | 29.6±6.7 | 29.8±10.8 |
| **kidney** | 10.1±2.1 | 8.6±1.9 | | 9.8±3.3 | 8.0±3.0 |
| **liver** | 8.7±2.1 | 14.0±4.4 | | 8.7±3.6 | 13.4±5.6 |
| **tumor/** | 0.2±0.1 | 1.0±0.3 | | 0.2±0.1 | 0.9±0.2 |
| **blood** | | | | | |
| **ratio** | | | | | |

| | | | | | |
|---|---|---|---|---|---|
| Mean ± S.D. N=5 animals/experimental group. | | | | | |

15% of the injected dose of radioactively labeled liposomes was still present in the circulation in both mouse models at 24 h after injection.

Approximately 5-10% of the injected dose could be recovered from tumor tissue in both models. Approximately the same amount was present in the livers. Relatively low amounts of liposomes were recovered from spleen, kidney and lungs.

### Example 3 - Tumor growth inhibition

B16F10 melanoma bearing mice received a single intravenous injection of an indicated dose of free pravastatin or liposomal pravastatin at the time when the tumor became palpable. At 7 days after treatment, tumor size was measured and tumor volume calculated according to the equation V = 0.52 x a² x b, wherein a is the smallest and b is the largest superficial diameter.

B16F10 melanoma bearing mice received a single intravenous injection of an indicated dose of free caffeic acid or liposomal caffeic acid at the time when the tumor became palpable. At 7 days after treatment, tumor size was measured and tumor volume calculated according to the equation V = 0.52 x a² x b, wherein a is the smallest and b is the largest superficial diameter.

To compare the effect of different doses of free pravastatin or liposomal pravastatin on tumor growth, B16-tumor bearing mice received a single injection of either formulation at the moment that the tumor became palpable. At 1 week after injection tumor volume was smaller with liposomal pravastatin. See the results in Fig. 1 wherein curve C is an untreated control.

The results for caffeic acid are shown in Figure 2.

## Claims

1. Use of a long-circulating colloidal carrier composition, such as liposomes, nanocapsules and polymeric micelles, comprising a natural or synthetic non-steroidal anti-inflammatory compound, for the manufacture of a medicament useful in the treatment of cancer.

2. The use of claim 1, wherein the medicament is useful in the treatment of non-lymphatic cancer, and especially in the treatment of solid primary and/or secondary tumors.

3. The use of claim 1 or 2, wherein the anti-inflammatory compound is selected from the group consisting of
(a) salicylates (like aspirin, methyl salicylate, diflunisal, benorylate, faislamine, amoxiprin);
(b) arylalkanoic acids (like diclofenac, indometacin, sulindac, 2-arylpropionic acids);
(c) profens (like carprofen, fenoprofen, flurbiprofen, ibuprofen, ketoprofen, ketorolac, loxoprofen, naproxen, tiaprofenic acid);
(d) N-arylanthranilic acids (like fenamic acids, mefenamic acid, meclofenamic acid);
(e) pyrazolidine derivatives (like phenylbutazone, oxyphenylbutazone);
(f) oxicams (like piroxicam, meloxicam);
(g) coxibs (like celecoxib, rofecoxib, valdecoxib, parecoxib, etoricoxib); sulphonanilides (like nimesulide);
(h) lipoxygenase inhibitors (like baicalein, caffeic acid, esculetin, gossypol, nordihydroguaiaretic acid, flurbiprofen, nordihydroguaiaretic acid, eicosatriynoic acid, 5- hydroxyeicosatetraenoic lactone, 5(S)-HETE, eicosatetraynoic acid);
(i) macrolide derivatives (like 9-(S)-dihydroerythromycin derivatives);
(j) natural compounds with anti-inflammatory properties like genistein, kaempferol, quercetin, resorcinol, resveratrol, docosahexaenoic acid, daidzein, epigallocatechin gallate (EGCG), alpha-tocopherol, ellagic acid, chlorogenic acid, p-coumaric acid, phytic acid, ferulic acid, vanillin, cinnamic acid, hydroxycinnamic acid, anthocyanins, catechins, isoflavones, hesperetin, naringin, rutin, silymarin, tangeretin, tannins, lycopene, lutein, saponins, terpeneol, terpene limonoids, lignans, or in general compounds belonging to the classes of terpenoids (isoprenoids), polyphenolics, glucosinolates, thiosulfonates, phytosterols, anthraquinones, capsaicins, piperines, chlorophylls, betaines, pectines, or oxalic acids; and (p) statins

4. The use according to claim 3, wherein the anti-inflammatory compound is selected from the group consisting of the poly(phenols) and statins.

5. The use according to any one of the preceding claims, wherein the colloidal carrier composition is a liposome, nano-capsule or a polymeric micelle, which colloidal carrier composition has a neutral or negative charge at physiological conditions.

6. The use according to any one of the preceding claims, wherein the colloidal carrier compositions are liposomes comprising a non-charged vesicle-forming lipid, 0-20 mole percent of an amphipathic vesicle-forming lipid derivatised with polyethyleneglycol, 0-50 mole percent of a sterol, and 0-10 mol % of a negatively charged vesicle-forming lipid, which liposomes have a selected mean particle diameter in the size range between about 40 - 200 nm.

7. The use according to any one of the preceding claims, wherein the colloidal carrier composition has a circulation half-life of at least 6 hours.

8. The use according to any one of the preceding claims, wherein the medicament is a medicament for parental or local application.

9. Pharmaceutical composition comprising non-steroid anti-inflammatory compounds, encapsulated in colloidal carriers, as defined in any one of the preceding claims.
